# EUROPEAN PATENT APPLICATION

(11) **EP 3 232 197 A1**
(43) Date of publication of application: **18.10.2017**
(21) Application number: 16165547.7
(22) Date of filing: 15.04.2016
(51) Int. Cl.: G01N 33/543, G01N 33/569

(54) **A NOVEL SUBSTRATE AND DEVICE FOR DETECTING AN ANALYTE AND METHOD FOR RAPID DIAGNOSIS**

(71) Applicant: LIONEX Diagnostics and Therapeutics GmbH, 38126 Braunschweig (DE)
(72) Inventor: SINGH, Mahavir, 38126 Braunschweig (DE); TRUTHMANN, Katja, 38126 Braunschweig (DE); MAYER, Anika, 38126 Braunschweig (DE)
(74) Representative: Kröncke, Rolf

(57) **Abstract**

The present invention relates to a, non-porous test-strip consisting of plastic sheet or a plastic hollow body as a substrate without any lateral or vertical flow of liquid through it. Further, the present invention relates to a device for detecting an analyte, in particular, for rapid testing for the presence of an analyte including a test strip according to the present invention. In addition, a device for rapid testing for the presence of an analyte is provided, said device comprises a test strip according to the present invention being part of or forming a hollow body with an inlet and an outlet for introducing and removing fluid therefrom and whereby the inner surface of the hollow body comprises the pre-determined area present on the test strip enabling immobilizing a reaction partner able to form a reaction product with the analyte to be determined. In addition, a method for rapid diagnosis of the presence of molecules of interest, namely, analytes, are provided based on the test strip and device according to the present invention.

## Description

The present invention relates to a non-porous test-strip consisting of plastic sheet or a plastic hollow body as a substrate without any lateral or vertical flow of liquid through it. Further, the present invention relates to a device for detecting an analyte, in particular, for rapid testing for the presence of an analyte including a test strip according to the present invention. In addition, a device for rapid testing for the presence of an analyte is provided, said device comprises a test strip according to the present invention being part of or forming a hollow body with an inlet and an outlet for introducing and removing fluid therefrom and whereby the inner surface of the hollow body comprises the pre-determined area present on the test strip enabling immobilizing a reaction partner able to form a reaction product with the analyte to be determined. In addition, a method for rapid diagnosis of the presence of molecules of interest, namely, analytes, are provided based on the test strip and device according to the present invention.

The plastic sheet or the plastic hollow body may be activated by any means such as by plasma treatment or wet by activation techniques, which are state of the art for coupling molecules of interest. The present invention further relates to the use of the plastic sheet or the plastic hollow body for developing several types of diagnostic tests, not requiring any lateral or vertical flow of the liquid. In addition, the present invention describes a novel type of rapid test for the detection of molecules of interest.

### Prior art

Today, billions of the so called lateral-flow-immunochromatographic rapid tests are manufactured and sold world-wide. The basic principles of the lateral flow technology have been firmly established since the beginning of the 1980s with the filing of several major patents by companies such as Beckton Dickinson & Co and Unilever and Carter Wallace (US 4703017, US 4855240 and US 5622871). The application of this technology has expanded well beyond clinical diagnostics to areas as diverse as agriculture, bio-warfare, food, environmental health, molecular diagnostic, therapeutic and veterinary field.

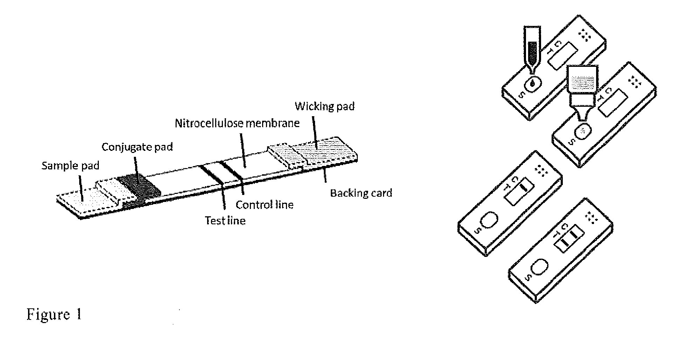

The basic design of all lateral flow tests consists of a membrane, usually nitrocellulose which is supported by a backing card. Several overlapping pads (sample pad, the conjugate pad, the wick) are assembled together allowing flow of liquid from the sample well to the absorbent wick at the other end (Fig. 1). The fundamental property of the test is the capillary flow of the liquid through the membrane which enables the reaction between the capturing molecule and the analyte.

Over the past decade substantial advances have been achieved in the *in-vitro* immunological diagnosis of *Mycobacterium tuberculosis* infection (Machingaidze S. et al., Pediatr Infect Dis. J. 2011; 30:694-700). Current diagnostic tests for tuberculosis (TB) diseases are either slow, expensive or unreliable. For example, the three commercially available tests based on specific interferon gamma release, namely, Interferon-gamma release assays (IGRAs) require contacting cells with specific mycobacterial antigens and, subsequently, detecting interferon gamma release by mononuclear cells or whole blood by ELISA requiring several days. Further, tuberculin skin tests (TST) are available. Said TST are based on the detection of a delayed type hypersensitivity response to an intradermal administration of a purified protein derivative of the mycobacterium. In adults, IGRAs have been reported to be more specific and at least as sensitive as TST and are currently included in diagnostic algorithms in adult guidelines. However, reported IGRAs are unable to differentiate between millions of infected but otherwise healthy individuals from the real TB patients suffering from active disease. The IGRA and TST are cumbersome tests requiring days and or special laboratory facilities. Sensitivity and specificity of IGRA largely vary among the studies in paediatric populations and caution is recommended regarding the use in children because of poor and contradicting data (e.g. Machingaidze, see above or Mandalakas AM. et al., Int J Tuberc lung Dis. 2011; 15:1018-32).

Alternative methods for determining tuberculosis include the determination of antibody profiles characteristic of tuberculosis. For example, antibody profiles are determined. US 2007/0026473 A1 describe antibody profiles directed against antigens (ESAT-6, 38kDAg, 16kDAg, Rv2626c, ferridoxine and alanine dehydrogenase).

The global public health and economic impact of infection with *Mycobacterium tuberculosis* and the closely related *Mycobacterium bovis* is severe. Approximately 9.8 million new cases of *M. tuberculosis* (MTB) infections were estimated for 2010 and, in addition, recent years have seen a rapid increase in *M. bovis* infections as well. Until today about 2 million people die annually due to TB. A rapid increase in the resistance against active compounds for the treatment of TB can be observed. For example, the prevalence of multidrug resistance (MDR) strains increases whereby MDR identifies resistance against at least the antibiotic compounds Isoniazid and Rifampicin. In addition, TB strains have been described being resistant against fluoroquinolon as well as capreomycin, kanamycin and Aminkacin beside the resistance against Isoniazid and Rifampicin. These strains are also identified as XDR-TB strains (extensively drug resistance strains).

There is an urgent need for providing sensitive and rapid diagnostic tools allowing diagnosis of TB for detecting early and/or latent infection with TB accordingly. However, as identified above, most of the diagnostic tests and systems require long duration and/ or laboratory facilities and are cost intensive. Thus, these systems are in particular not usable in the third world where a cheap, reliable and fast diagnostic test is required. That is, in particular in the developing countries it is essential to diagnose TB at an early time point using simple test systems based on detection of antigens or antibodies specific for TB. In addition, for providing rapid test kits to be useful in developing countries, it is desired to use the test directly with body fluid, in particular, blood without requiring time consuming and costly steps such as culture, IGRA and target amplification such as in PCR, GenXpert (http://www.cepheid.com/). That is, complex and cost intensive detection systems based on molecular approaches, like PCR or tests based on cultivation and detection of cytokines (IGRAs) cannot be used in developing countries. Tuberculin tests may result in false positive results due to BCG vaccination in advance. Microscopic detection is less sensitive.

The aim of the present invention is to provide novel platform for developing cheap and effective rapid tests, for example, for 1. tuberculosis, 2. other human diseases, 3. food industry, 4. environmental field, 5. Bio-warfare security. Said tests and devices should be useful also in developing countries, thus, a requirement is an economical test with simple and easy handling of said devices and test systems and no use of any carrier material so that laymen can use the test in developing countries accordingly.

### Summary of the present invention

This invention presents a novel technology drastically different from the Lateral Flow Immunchromatographic test since no flow of liquid is required. The simplest version is named as LIODROP^{®} and is presented here as an example of a novel test for the diagnosis of tuberculosis, a global diseases in humans claiming the maximum number of deaths caused by any single bacterial pathogen.

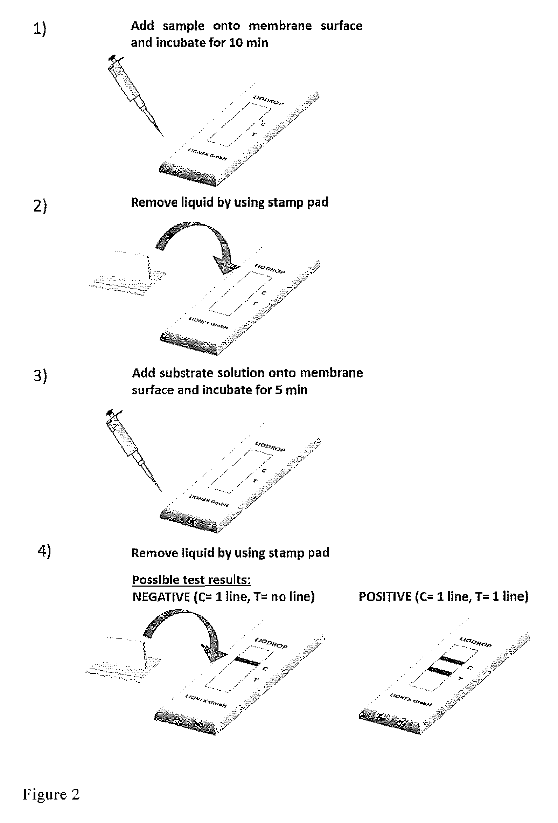

In a first aspect, the present invention provides a test strip for detecting an analyte composed of a non-porous synthetic sheet, in particular, plastic sheet, having at least one predetermined area with an immobilized reaction partner able to react with the analyte including binding the analyte to be determined, for forming a reaction product, like a binding pair whereby the predetermined area is treated before immobilizing the reaction partner able to react with the analyte to be determined. (Fig. 2).

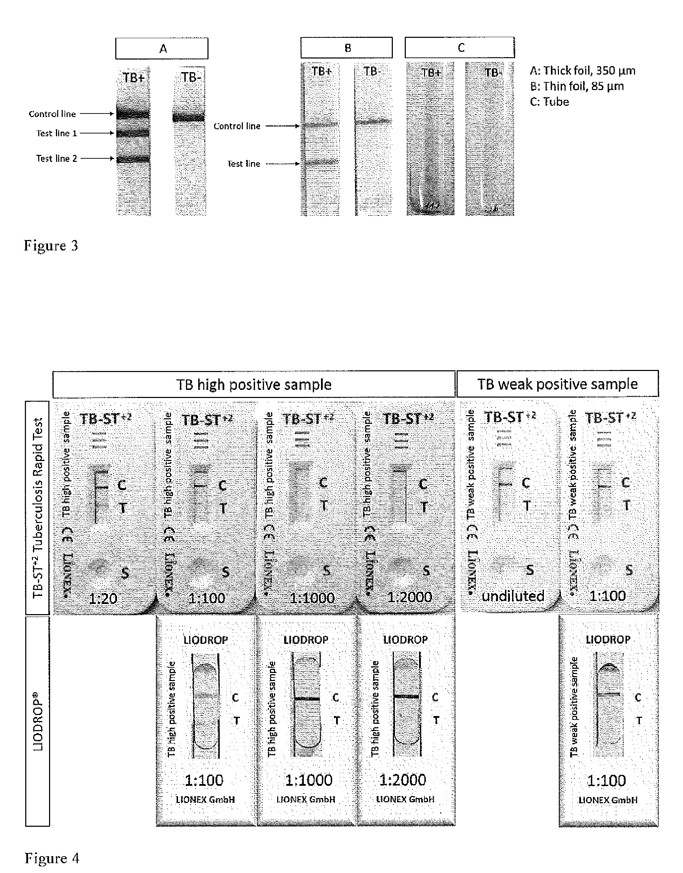

In its simplest version, the plastic sheet can be used without any support. Other versions could be where a cassette is used to fix the plastic sheet which is favoured in case of thin flexible plastic sheets (Fig. 2). Additional versions of plastic sheet could be in the form of a tube which is coated with the capture molecule in the form of a ring or lines (Fig. 3). In this case, certain amount of the fluid to be analysed is added to the tube so that the coated surface is dipped in the fluid containing the analyte. Such tube format is highly suitable for analysing samples which are available in larger volumes such human urine or water from environmental sources.

As used herein, the terms "test strip" and "plastic sheet" are used interchangeably unless otherwise indicated.

Further, the terms "synthetic sheet" and "plastic sheet" are used herein interchangeably unless otherwise indicated.

Moreover, in a further aspect, the present invention relates to a device for rapid testing for the presence of analytes, in particular, disease specific antigens or disease specific antibodies comprising a plastic sheet having a thickness of 0.5 mm or less.

In a further aspect, the present invention relates to a device for detecting an analyte comprising the test strip according to the present invention, e.g. in form of a plastic sheet, a housing, and means for fixing the test strip, said housing determine a first spot of introducing a fluid to be analysed on test strip, the test strip is fixed with the means, thus, forming an inclined plane sloping downward from the spot of introducing the fluid to be analysed at the upper region to the lower region of the test strip thereby allowing flowing of said fluid over at least one predetermined area on the test strip.

Alternatively, the test strip is essentially horizontal and a drop of fluid containing the analyte to be analysed is added onto the test strip and kept for a predetermined time.

In a further aspect, the present invention relates to a device for rapid testing for the presence of analytes, in particular, disease specific antigens or disease specific antibodies comprising a test strip according to the present invention being part of or forming a hollow body with an inlet for introducing fluids and an outlet for removing the fluid from the hollow body, wherein the inner surface of the hollow body comprises a predetermined area having immobilized a reaction partner able to form a reaction product with the analytes to be determined.

Finally, the present invention relates to test systems including the devices according to the present invention.

The present inventors recognized that an affordable and rapid test system for diagnosing tuberculosis or other diseases can be provided. These devices are particular useful for allowing TB diagnosis in developing and third world countries.

Surprisingly, the devices according to the present invention do not need any further support or carrier material, thus, decreasing the costs of the tests and, in addition, the devices and test systems according to the present invention allows a rapid diagnosis for example in a drop of liquid. Moreover, the devices and test systems according to the present invention can be stored over a long period and under conditions of higher temperature as it is case in developing countries, in particular, in countries located in Africa.

Moreover, detection of hormones such as progesterone can be performed in body body fluids such as blood or serum or urine.

### Brief description of the drawing

Figure 1 shows the basic design of all lateral flow tests (test strip and cassette format).
Figure 2 shows the LIODROP^{®} test principle.
Figure 3: 3A shows an example of a thick sheet showing two positive test lines, 3B shows an example of a thin sheet showing one positive test line, 3C shows an example of a tube showing one positive vertical line.
Figure 4 shows a comparison of current test systems (TB-ST⁺² Tuberculosis Rapid Test) to the present invention.
Figure 5A shows strips coated with Tb specific antigen (test line) and control protein (control line).
Figure 5B shows strips coated with antigen + Human Sera Tb negative (healthy).
Figure 5C shows strips coated with antigen + Human Sera Tb negative (healthy) + anti-human-IgG-HRP.
Figure 5D shows strips coated with antigen + Human Sera Tb positive.
Figure 5E shows strips coated with antigen + Human Sera Tb positive + anti-human-IgG-HRP.

### Detailed description of the present invention

The present invention relates in a first aspect to a test strip for detecting an analyte, said strip being composed of a non-porous plastic sheet e.g. having a thickness of 0,5 mm or less, and having at least one predetermined area with immobilized reaction partner able to react with the analyte to be determined for forming a reaction product whereby the predetermined area is pre-treated, in particular, at least partly by a plasma treatment, before immobilizing the reaction partner able to form a reaction product with the analyte to be determined. Advantageously, the predetermined area is at least partly treated by plasma treatment. In contrast to assays and test systems, presently available, the test strip according to the present invention allows rapid and easy but reliable detection and diagnosis of e.g. TB (Fig. 4).

The term "strip" refers to any form of a sheet, including round, oval, elongated, etc.

In another embodiment of the present invention, the present invention relates to a device for detecting an analyte comprising a plastic sheet with a first spot of introducing a fluid to be analysed on the plastic sheet, the plastic sheet, if necessary, is fixed in the assembly forming an inclined plane sloping downward from the spot of introducing the fluid to be analysed at the upper region to the lower region of the plastic sheet thereby allowing flowing of said fluid over at least one predetermined area on the plastic sheet whereby the plastic sheet comprises at least one predetermined area with immobilized molecules able to bind the analyte to be determined.

It is preferred, that the analyte is an antigen with an epitope recognized specifically by antibodies, in particular, monoclonal antibodies. That is, it is preferred that the analyte according to the present invention is a marker molecule, in particular, a disease specific antigen present in the body fluid of an individual afflicted with or suspected to be afflicted with tuberculosis infection or other diseases, disorders or conditions.

Alternatively, the analyte is a disease specific antibody, in particular, an antibody specific for a MTB antigen.

As used herein, the term "individual" in the context of the present invention is typically a mammal, in particular, the mammal is a human, a non-human primate or other mammals, in particular, mammals susceptible for mycobacterial infection. The individual can be male or female.

The term "determining" or "detecting" as used herein refers to assessing the presence, absence, quantity, level or amount of either given substance within a clinical or subject derived sample, including qualitative and/or quantitative concentration levels of substances otherwise evaluating values or categorizing of a subject clinical parameter.

The term "comprises" or "comprising" or "contains" or "containing" includes the embodiments of "consist" or "consisting".

The term "at least partly" include the embodiment of full.

The term "plastic sheet" includes embodiments of plastic sheet. For example, the plastic sheet is a cling film or rapping film. The plastic sheet is composed e.g. of PP, PVC or PE or other adhesive sheets. Other types of plastic may be used including low density polyethylene (LDPE). In an embodiment of the present invention, the plastic sheet or plastic sheet may be a non-porous sheet. That is, the fluid placed on the plastic sheet can remain on the sheet or flow downwards when the plastic sheet is sloping downward from the spot of introducing the fluid at an upper region of the plastic sheet to the lower region of the plastic sheet. Rather, the fluid to be analysed may be placed directly on the predetermined area, thus, the fluid can flow through the sheet while the analyte to be determined can bind to the immobilized binding partner present in the predetermined area.

Of course, it is possible to use non-porous plastic sheets in a device wherein the plastic sheet is inclined as well.

In another embodiment, the present invention relates to a device for rapid testing for the presence of analytes, in particular, disease specific antigens or disease specific antibodies, comprising a hollow body with an inlet for introducing fluids and an outlet for removing the fluid from the hollow body, wherein the inner surface of the hollow body is activated, in particular, treated at least partly by plasma treatment, and wherein a predetermined area of the inner surface of the hollow body being activated, in particular, pre-treated by plasma treatment, have immobilized a binding partner able to form a binding pair with the analytes to be determined.

The material of the plastic sheet or the hollow body is typically plastic material known in the art. While for the plastic sheet or plastic sheet a suitable material is one of the materials described above, the hollow body may be a plastic material composed of PS, PP or PE as well as PVC.

A possible embodiment of the hollow body with the inlet and outlet is a syringe.

For example, commercially available syringes may be used as a hollow body and may be prepared to be a device according to the present invention. For example in case of a syringe being a device for rapid testing for the presence of analytes according to the present invention, the fluid containing the analyte to be tested enters the hollow body through the at least one opening allowing introduction and removal of the fluid from the hollow body.

A preferred embodiment of the fluid to be analysed is a body fluid, in particular, urine, blood including serum or plasma or sputum, or other fluids from the environment or veterinary sources.

In an embodiment of the present invention, the test strip or the device according to the present invention does not contain any additional carrier, in particular, not having any additional porous carrier or additional membrane. That is, in contrast to known devices for lateral flow application, the fluid does not flow through any carrier or membrane, for example by capillary forces but the fluid stays on the sheet.

That is, the devices can be handled easily, can be produced cheaply and, in addition, allow to be used by any unskilled person.

The analyte to be analysed and the binding partner immobilized on the plastic sheet or on the inner surface of the hollow body may form a binding pair. Typical examples of binding pairs include antibody/antigen complex; ligand/receptor complex.

In an embodiment of the present invention, the binding pair is an antigen/antibody complex. In an embodiment, the antibody, in particular, a monoclonal antibody is immobilized on the plastic sheet or on the inner surface of the hollow body while the analyte to be determined is an antigen binding specifically to said antibody.

In another embodiment, the antigen is immobilized on the plastic sheet or the inner surface of the hollow body while antibodies present in the fluid which bind specifically to the immobilized antigens, are bound.

The skilled person will select suitable binding pairs, in particular, will select a suitable binding partner to be immobilized on the plastic sheet or at the surface at the hollow body enabling specific binding of the analyte to be analysed and, subsequently, detection of the binding pair for example by an enzymatic reaction.

In case of a device comprising a plastic sheet, said device may have additionally a residue space for storing the residual fluid, like the liquid. In case of a device according to the present invention having an inclined plastic sheet, said residual space may be arranged at the lower region of the plastic sheet. In case of an essentially horizontal non-porous plastic sheet, the residual space may be located underneath said test strip.

In an embodiment, said residual space underneath said porous plastic sheet or at the lower region of the plastic sheet contains moisture absorbent material. In an embodiment, the plastic sheet or the surface of the hollow body has at least two predetermined areas whereby e.g. one predetermined area represents a control.

In case of at least two predetermined areas, each predetermined area may contain a different binding partner, thus, allowing binding of different analytes. The binding pairs are selected in a way allowing specific formation of binding pairs and no cross reaction between analytes to be determined and different binding partners present in different predetermined areas.

The device according to the present invention have fixing means for fixing the test strip in the housing of the device. The skilled person is well aware of suitable fixing means accordingly.

The predetermined area for immobilizing the binding partner are activated in advance before incubation with the binding partners to be immobilized.

The skilled person is well aware of suitable methods for activation. For example, activation may be conducted by atmosphereic plasma treatment, low pressure plasma treatment, wet treatment among others.

A suitable embodiment for activation is plasma pre-treatment. For example, suitable methods are described in EP 2046506 B1 also published as WO 2008/014915. This kind of plasma pre-treatment allows surface body preactivation, e.g. by gas plasma using suitable carrier gases and suitable reactions, thus, providing a suitable process gas for activating the predetermined areas.

Cold plasma may be used for surface modification. In particular, a suitable method for activation of the surface of the plastic sheet or the inner surface of a hollow body is a cold plasma method at atmospheric pressure using suitable process gases allowing the formation of suitable functional groups present on the surface of the plastic. These functional groups represent binding sides for the binding partner.

For example, the plasma pre-treatment may be effected specifically, thus, forming predetermined areas, e.g. dots or stripes for later immobilization of specific binding partners accordingly.

In a preferred embodiment, the test strip or the device according to the present invention is a test strip or a device suitable for determining infection with mycobacterium, in particular, *Mycobacterium tuberculosis.* That is, the binding pair is typically an antibody/antigen complex formed from antigens derived from mycobacterium, like *Mycobacterium tuberculosis* specific molecules, and the antibodies are antibodies binding specifically to mycobacterial antigens. In an embodiment, monoclonal antibodies are immobilized as binding partners while the analytes to be analysed are antigens, in particular, mycobacterial antigens.

In an embodiment, the test strip or the device according to the present invention contain at least two different predetermined areas having immobilized different reaction partners, like binding partners, e.g. different monoclonal antibodies being immobilized at predetermined activated, in particular, plasma pre-treated areas in the inner surface of the hollow body whereby the at least two different predetermined areas with immobilized different reaction partners, e.g. binding partners, like monoclonal antibodies being specific for different mycobacterial antigens allowing for determination of different analytes, like different mycobacterial antigens.

In another aspect, the present invention relates to test systems comprising the test strip or the device according to the present invention. These test systems are in particular diagnostic test systems or diagnostic kits suitable for diagnosing of analytes representing marker molecules specific for a disease, disorder or condition. For example, these marker molecules are molecules representing mycobacterial infection, like *mycobacterial tuberculosis* infection. That is, the analytes are e.g. mycobacterial antigens or antibodies directed against mycobacterial antigens and the test system according to the present invention allows to determine the presence thereof accordingly. Thus, it is possible to diagnose mycobacterial, like MTB, infection and TB diseases.

The test system according to the present invention may contain additional components. These additional components include washing buffers, as well as detection means for detecting the formation of the binding pair.

In this connection, the term "binding pair" as used herein refers to a complex of at least two binding molecules also referred to as binding moieties, namely a first moiety of the binding pair and a second moiety of the binding pair. For example, a binding pair is formed of an antibody and the antigen.

The skilled person is well aware of suitable detecting means. For example, said detecting means are monoclonal antibodies or aptamers binding to the analyte.

In addition, the test system contain components allowing visualization of the binding pairs, for example using a marker component. Said marker component may be any one of marker components known in the art and including the use of radioactive isotopes, fluorescent molecules, enzymatic activity, peptide tasgs, etc.

In a further embodiment, the present invention relates to a method for rapid diagnosis of the presence of analytes, like of marker molecules, comprising the steps of:
- providing a test strip or a device according to the present invention;
- introducing a sample supposed to contain the analytes into or onto the test strip or device for allowing reaction of the analytes with the reaction partner immobilized at predetermined areas of the test strip of the device for forming a reaction product;
- reaction, like binding, of the analytes to the reaction partner forming a reaction product including a binding pair; and
- determining the presence of the reaction product including the binding pair composed of the analyte and the reaction partner.

In a further embodiment, the present invention relates to a method for rapid diagnosis of the presence of analytes, like of marker molecules, comprising providing a device or a test system or test kit, in particular, a diagnostic test system as described herein; introducing a sample supposed to contain the analyte to be analysed into the device or the hollow body for allowing binding of the analytes to a binding partner immobilized at a predetermined area in the hollow body or the plastic sheet of the device, thus, forming a binding pair; binding of the analytes to the binding partner forming a binding pair; and determining the presence of the binding pair composed of the analyte and the binding partner.

In an embodiment, detection of the binding pair comprises contacting the analyte bound to its binding partner forming the binding pair with a detecting agent for detecting the presence of the binding pair.

For example, detection may be conducted by colour reaction using horse-radish peroxidase or alkaline phosphatase and their corresponding substrates.

In an embodiment of the method according to the present invention, the presence of the binding pair in the predetermined area is indicative for the presence of the analyte in the sample.

The method according to the present invention using the test strip, the devices and test systems according to the present invention allow a rapid and reliable test for identifying and diagnosing a disease, disorder or condition based on marker molecules, in particular, allow to diagnose mycobacterial infection, in particular, *Mycobacteria tuberculosis* infection. Based on the marker molecules and antibody used, it may also be possible to determine the tuberculosis state of the individual to be tested. That is, the rapid test system allows to diagnose for mycobacterial infection and, dependent on the binding partners immobilized on the surface, allows to determine the status of tuberculosis infection in the individual including distinguishing between active and latent tuberculosis infection. This method is particularly useful in developing and third world countries to identify at an early time point a possible infection. That is, when applying a method wherein disease specific antigens present in body fluids from patients are identified, in particular, in blood, whereby detection is possible by using monoclonal antibodies being specific to said antigens, it is possible to determine an infection independently from the immune status of the individual. For example, the method include the use of monoclonal antibodies being specific against the following antigens: M. tuberculosis AlaDH; Rv2780; M. tub. 16 kDa antigen (Rv2031c); M. tub. MPT64 (Rv1980c); proteins of the 85 kD complex (85A (Rv3804c), 85B (Rv1886c) and 85C (Rv0219c)), furthermore, useful antigens are Esat6 (Rv3875) and M. tub. CFP10 (Rv3874). Further, for example, when using urine as body fluid, the following antigens may be detected Rv1656, Rv1681, M. tub. cysH (Rv2392) and Rv3341.

The test system itself may be a flow through test system wherein some fluids may stay in the system for a while due to a fastening element at the at least one opening. In case of a device having two openings, namely, a first opening for introducing a fluid and a second opening for removal of said fluid, the device may be used as a run through system including the washing and detection steps. However, the test system is not a lateral flow system based on an immunochromatographic method.

Due to the immobilization of the binding partner directly on the surface of the plastic material, no additional carrier material is required, thus, reducing the production costs.

In case of a hollow body, the predetermined areas may be provided having different structures. For example, the predetermined areas may be present as horizontal areas one above the other. Figure 3 shows an example of a hollow body (tube) having in the inner surface various predetermined areas which have different immobilized binding partners.

Alternatively, the predetermined areas are arranged vertically at the inner surface of a hollow body. Figure 3 shows an example of predetermined areas. Said predetermined areas have immobilized a binding partner able to form a binding pair with analytes to be determined. Typically, each predetermined area has a different binding partner, thus, allowing a multiplex analysis accordingly.

In case of using a syringe or other suitable hollow bodies, the body fluid, like urine, blood or sputum is filled into the syringe and the body fluid can stay for a predetermined time therein, typically, the time is 20 minutes or less. Thereafter, washing steps are applied as well as steps allowing detection of binding pairs typically, by applying known methods for colour detection.

In the following, suitable embodiments are described further by referring to the figures. Figure 2 and 4 shows a device according to a first embodiment of the present invention.

The present invention will be described by way of examples further without limiting the same thereto.

### Examples

### Example 1 Activation of plastic sheet

Foundation of coupling chemistry of 2D substrates on the surface of functionalized plastic sheets (thin sheets, thick sheets, PP, PVC, a.s.o.). Different plasma coating processes were analysed. Processes like aldehyde group functionalization or carboxylation were used to intense the protein binding of patented TB antigens or antibodies onto the plastic sheet surface.
To investigate the protein binding, the plastic sheets were coated with the atmospheric pressure plasma method using (3-aminopropyl) trimethoxysilane (APTMS). As precursor GMA was used. The flow rates were varied. The carrier gases were usually Ar, N₂, H₂.

### Example 2 Activation of the inner surface of a hollow body

Foundation of coupling chemistry of 2D substrates on the surface of functionalized 3D substrates (such as tubes, syringes). During plasma coated process aldehyde groups are functionalized on 3D substrates to intense the protein binding of patented TB antigens or antibodies.
To investigate the protein binding of aldehyde, the plastic syringes or tubes were coated with the atmospheric pressure plasma method using (3-aminopropyl) trimethoxysilane (APTMS). As precursor GMA was used. The flow rates and carrier gases were varied.

### Example 3 Immobilization of a binding partner

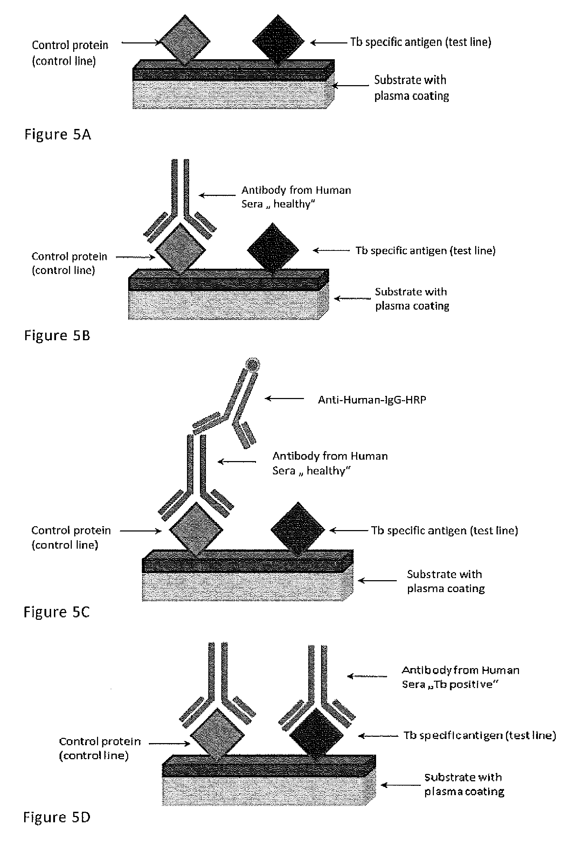

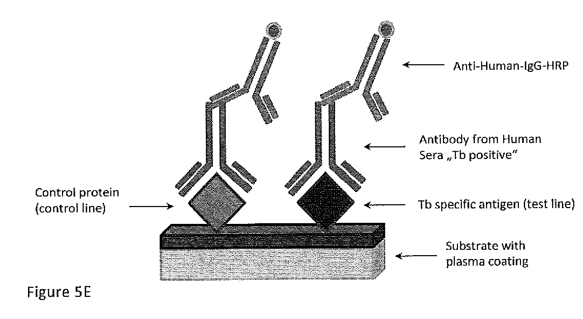

The aim of the present inventors was to develop a new test system for the detection of different Tuberculosis antigens in a new designed rapid test with higher sensitivity to standard lateral flow tests. A special type of plasma coated sheets should replace the often used nitrocellulose in a lateral flow test. The advantage of using plasma coated sheets is that an enzymatic test could be developed which is much more sensitive than the detection with colloidal gold. (Fig. 5A-E).

### Example 4 Detection of antigen specific binding pairs

The test development with plastic syringes was performed by coating with Tb specific antigen or antibody (10 minute incubation with coating solution during rotating conditions). The coating solution was removed and the syringe dried at 37°C for approx. 20 minutes. The sample containing the related antigen or antibody was added into the syringe and incubated for 10 minutes at room temperature during rotating conditions. After this, the sample was removed and the antigen or antibody was detected via biotin-labelled antibody plus HRP-conjugated Streptavidin mixture (10 minute incubation at room temperature during rotating conditions). After this, two washing steps with 1x TBS-Tween (0.05%) for removing unbound material has to be done and a final incubation with TMB (10 minutes in the dark) develops to blue signal, if the sample contained related antigens/antibodies.

Using a cling film (plastic wrap): 1 µL of antibody spotted onto surface, as negative control PBS, pH 7.4 was used. The liquid spots were dried at 37°C for approx. 30 minutes. The cling film was blocked by applying 200 µL of 1% BSA solution in PBS, pH 7.4 for a couple of seconds and removed by using Whatman paper pad. The cling film was dried again at 37°C for about 5 minutes and 100 µL of HRP-conjugated antibody (diluted in 1% BSA solution in PBS, pH 7.4) was applied for detection. The solution was incubated for 20 minutes and removed by using a Q-Tip. After this, 100 µL of TMB solution was added and incubated for 10 minutes at room temperature in the dark. The TMB solution removed by using Q-Tip and the results were documented immediately.

## Claims

1. A test strip for detecting an analyte, said test strip being composed of a non-porous synthetic sheet having at least one predetermined area with immobilized reaction partner able to react with the analyte to be determined for forming a reaction product whereby the pre-determined area is pre-treated, in particular, at least partly pre-treated by a plasma treatment, before immobilizing the reaction partner able to form a reaction product with the analyte to be determined.

2. The test strip for detecting an analyte according to claim 1 composed of a plastic including polyvinylchloride (PVC), polypropylene (PP), polyethylene (PE), polycarbonate (PC), low density poly-ethylene (LDPE) and mixtures thereof, in particular, composed of polyvinylchloride.

3. The test strip for detecting an analyte according to claim 1 or 2 comprising further at least a second pre-determined area whereby at least one of the pre-determined areas represents a control.

4. The test strip for detecting an analyte according to any one of the preceding claims wherein the analyte and the immobilized reaction partner are immunological reaction partners forming a binding pair of an antigen-antibody-complex or receptor-ligand complex.

5. The test strip for detecting an analyte according to any one of the preceding claims wherein the immobilized binding partner are disease specific antigens, in particular, antigens derived from *Mycobacterium tuberculosis* specific molecules.

6. The test strip according to any one of the preceding claims containing at least two different predetermined areas having immobilized different binding partner being immobilized in predetermined plasma pre-treated areas allowing for determination of different analytes.

7. A device for detecting an analyte, in particular, for rapid testing for the presence of analytes, in particular, disease specific antigens or disease specific antibodies, comprising a test strip according to any one of claims 1 to 6, a housing and means for retaining said test strip within said housing.

8. A device for detecting an analyte, in particular, for rapid testing for the presence of analytes, in particular, disease specific antigens or disease specific antibodies, comprising a test strip according to any one of claims 1 to 6 being part of or forming a hollow body with at least one opening allowing introduction and removal of the fluid from the hollow body, wherein the inner surface of the hollow body comprises the predetermined area having the immobilized reaction partner able to form a reaction product with the analytes to be determined.

9. The device according to any one of claims 7 or 8 not containing any additional carrier, in particular, not having any porous carrier or membrane.

10. The device according to any one of the preceding claims 7 to 9 having additionally a residual space for storing residual liquid arranged at the lower region of the plastic sheet test strip.

11. The device according to claim 10 wherein said residual space at the lower region of the plastic sheet test strip contains moisture absorbent material.

12. A method for rapid diagnosis of the presence of analytes, like of marker molecules, comprising the steps of:
- providing a test strip or a device according to any one of claims 1 to 11;
- introducing a sample supposed to contain the analytes into or onto the test strip or device for allowing binding reaction of the analytes with device for forming a reaction product reaction, like binding, of the analytes to the binding reaction partner;
- forming a reaction product including a binding pair; and
- determining the presence of the reaction product including the binding pair composed of the analyte and the binding reaction partner.

13. The method according to claim 12 wherein detection of the binding pair comprises contacting the analyte bound to the binding partner in the binding pair with a detection agent for detecting the presence of the binding pair.

14. The method according to any one of claims 12 to 13 wherein the presence of the reaction product including the binding pair in the predetermined area is indicative for the presence of the analyte in the sample.

15. The method according to any one of claims 12 to 14 where the sample is a body fluid, like blood, urine, sputum, saliva, cerebrospinal fluid or brochoalveolar lavage, or any liquid of environmental source, like water or sewage.
